# EUROPEAN PATENT APPLICATION

(11) **EP 1 833 020 A2**
(43) Date of publication of application: **12.09.2007**
(21) Application number: 07250758.5
(22) Date of filing: 22.02.2007
(51) Int. Cl.: G06T 7/00, G06T 11/00, A61B 6/00

(54) **Attribute based image enhancement and display for medical imaging applications**

(30) Priority: 09.03.2006 US 780605 P; 09.03.2006 US 780748 P; 01.05.2006 US 414842
(71) Applicant: iCad, Inc., Beavercreek, Ohio 45431 (US)
(72) Inventor: Dolwick, Terry, Enon, Ohio 45323 (US); Worrell, Steve, Beavercreek, Ohio 45431 (US); Knapp, Jason Forrest, Miamisburg, Ohio 45342 (US)
(74) Representative: Cummings, Sean Patrick

(57) **Abstract**

A method and system for enhancing the display of computer-aided detection marks on a medical image without obscuring underlying details are disclosed. A medical image is obtained and processed by a computer-aided detection (CAD) algorithm. The image is displayed or printed with its corresponding CAD-detected regions superimposed on the image. In one embodiment, the characteristics of the CAD-detected region will be enhanced in order to emphasis the suspicious image features. In another embodiment, a region surrounding the CAD-detection regions will be brighten with enhanced contrast by brightening the pixels associated with that region as compared to the rest of the image so that region surrounding the CAD-detected region clearly stands out. When viewed on a display, the user can toggle the CAD-detected region enhancements on or off. Further, the CAD-detected regions can be cropped from the medical image to further improve the visibility of image attributes.

## Description

### CROSS-REFERENCE TO RELATED APPLICATIONS

This application claims the benefit of U.S. Provisional Application Serial No. 60/780,605 (ICA 0103 MA), filed March 9, 2006, and U.S. Provisional Application Serial No. 60/780,748 (ICA 0105 MA), filed March 9, 2006.

### BACKGROUND OF THE INVENTION

The present invention relates generally to a method and system for enhancing and displaying image processing and pattern recognition of image regions on a medical image and, in particular, to a method and system of image processing and pattern recognition methods for enhancing and displaying image regions consistent with known a-priori patterns and/or observables of interest to a user.

Computer-aided detection (CAD) systems are becoming more widely used to assist readers, such as radiologists, of medical imagery. Such CAD systems are commercially available to assist radiologists in the detection of potential areas of concern such as, for example, signs of cancer in mammographic, colon and chest imagery by highlighting regions of possible malignancies. In essence, these CAD systems become a second reader of the medical imagery for the radiologist.

At a fundamental level, CAD system operation may be explained as the sequence of operations, comprising, in order, detection, discrimination, and display. In detection, input imagery is analyzed to locate candidate regions with characteristics typical of malignancies. The input imagery can be from a variety of sources including digitized film and systems which directly create digital images. Separate processing paths are typically provided to individually detect common types of cancer, such as, for example, the case of mammography: a mass detector and a clustered microcalcification detector.

In practice, typically many users quickly become accustomed to the operation of the CAD system. Therefore, most CAD system outputs can be readily interpreted by users. That is, a displayed detection is either accepted as malignant or is easily dismissed as a false positive mark due to some observable artifact in the image. CAD systems use complex image processing and pattern recognition algorithms to identify regions within a medical image consistent with known characteristics of cancers. Using an associated similarity metric, marks are generated via thresholding a similarity metric and burned into or overlaid on the image. Consequently, these marks alert the user to the regions of concern but, at the same time, also can obscure image detail. Furthermore, all regions of concern may not be marked due to the binary thresholding process.

By enhancing and/or de-emphasizing all pixels based on a similarity score, the disadvantages of only marking and enhancing discrete regions can be eliminated. Rather all pixel intensities in the image can be modulated in accordance to a similarity score, thus allowing users to detect more suspicious regions based on inspection of an enhanced image, even in the event that no secondary cue is provided. This method avoids relying exclusively on a thresholded similarity metric and subsequent use of binary annotations that can lead to unnecessary variability in annotations due to digitizer noise, projection effects, and exposure conditions as well as oversight of relevant regions due to absence of annotations.

Therefore, there is a need for a method of enhancing the region mark by the CAD system without obscuring the underlying medical image detail. In addition, by marking the surrounding areas less important, the user would also be alerted to the area without the deficiencies of the marks.

There is an additional need for a method of enhancing the region surrounding the area marked by the CAD system for the user to better understand why the computer algorithms of the CAD system marked a particular image region.

Finally, there is a need for a method to avoid setting a somewhat arbitrary binary decision threshold for the purpose of identifying discrete image regions for review.

### BRIEF SUMMARY OF THE INVENTION

According to the present invention, a method and system for enhancing the display of computer-aided detection findings marks on a medical image without obscuring the underlying details of the medical image is disclosed. A medical image is obtained and processed by a computer-aided detection algorithm. The image is displayed or printed with its corresponding CAD-detected regions superimposed on the image. The CAD-detected regions will displayed such that the characteristics of the CAD-detected region and the area surrounding the CAD-detected regions will be enhanced so as to give the user additional information concerning the CAD-detected region.

In accordance with one embodiment of the present invention, the characteristics of the CAD-detected region will be enhanced in order to over-emphasis the suspicious image features. For example, in the case of a spiculated mass, the linear structures could be emphasized.

In accordance with another embodiment of the present invention, a region surrounding the CAD-detection regions will be brighten with enhanced contrast as compared to the rest of the image so that surrounding region clearly stands out from the rest of the image.

In accordance with yet another embodiment of the present invention, when viewing the medical image on a workstation display, the user could toggle the CAD-detected region enhancements on or off on the medical image.

In accordance with still another embodiment of the present invention, the CAD-detected regions could be cropped out of the medical image and displayed separately so that the user can easily view the attributes associated with areas of interest.

Accordingly, it is a feature of the embodiments of the present invention to remove the impact of obscuring important image detail with an overlaid CAD system mark.

It is another feature of the embodiments of the present invention to tailor the type of CAD enhancement to assist the user in better understanding why the CAD system marked a particular image region.

It is yet another feature of the embodiments of the present invention to avoid setting a somewhat arbitrary binary decision threshold for the purpose of identifying discrete image regions for review.

Other features of the embodiments of the present invention will be apparent in light of the description of the invention embodied herein.

### BRIEF DESCRIPTION OF THE SEVERAL VIEWS OF THE DRAWINGS

The following detailed description of specific embodiments of the present invention can be best understood when read in conjunction with the following drawings, where like structure is indicated with like reference numerals and in which:

Fig. 1 illustrates a high level block diagram of the flow of data through the system according to an embodiment of the present invention.

Figs. 2A and B illustrate brightening a region surrounding a CAD-detected region of interest according to an embodiment of the present invention.

Fig. 3A-C illustrates brightening the pixels associated with the characteristics of a microcalcification CAD-detected regions of interest according to an embodiment of the present invention.

Fig. 4 illustrates the steps for detecting and enhancing a region with margins for a circumscribed mass according to an embodiment of the present invention.

Fig. 5 illustrates the steps for detecting and enhancing a region with spicules for a spiculated mass according to an embodiment of the present invention.

Fig. 6 is a flow chart illustrating the steps involved in a mass enhancement according to an embodiment of the present invention.

Fig. 7 is example of a single mass detection enhancement on a mammography image according to an embodiment of the present invention.

Fig. 8 is a flow chart illustrating the steps involved in a microcalcification enhancement according to an embodiment of the present invention.

Fig. 9 is an example of a single microcalcification detection enhancement on a mammography image according to an embodiment of the present invention.

Fig. 10 is an example of enhancements of a non-overlapping single microcalcification detection and a single mass detection on a mammography image according to an embodiment of the present invention.

Fig. 11 is an example of enhancements of a single microcalcification detection and a microcalcification detection on top of a mass detection on a mammography image according to an embodiment of the present invention.

Fig. 12 is an example of enhancements of a single mass detection and a microcalcification detection on top of a mass detection on a mammography image according to an embodiment of the present invention.

Fig. 13 illustrates enhancing a mass detection by padding or not padding the mass detection according to an embodiment of the present invention.

### DETAILED DESCRIPTION

In the following detailed description of the embodiments, reference is made to the accompanying drawings that form a part hereof, and in which are shown by way of illustration, and not by way of limitation, specific embodiments in which the invention may be practiced. It is to be understood that other embodiments may be utilized and that logical, mechanical and electrical changes may be made without departing from the spirit and scope of the present invention.

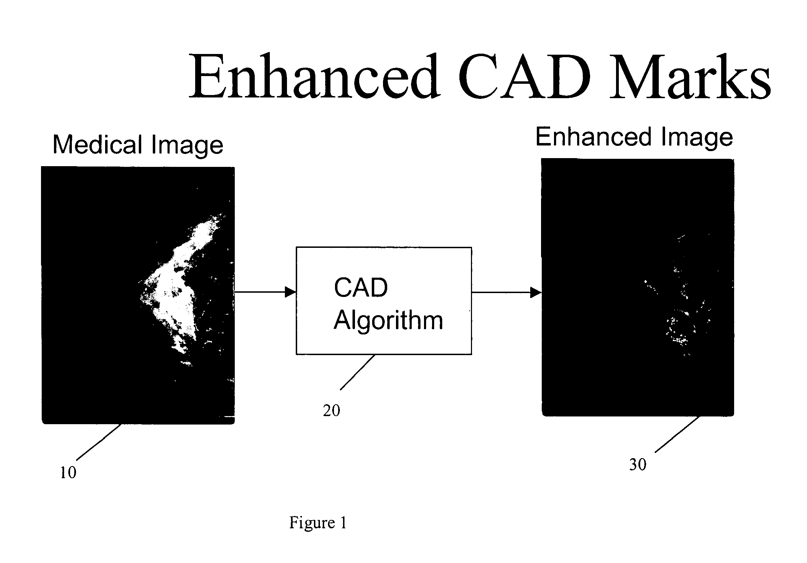

Referring initially to Fig. 1, a high level block diagram of the flow of data through the system is illustrated. A medical image, or a set of medical images, is obtained in step 10. The medical image can be, for example, a mammography X-ray image, a lung X-ray image, a colonoscopy image, or three dimensional data such as Computed Tomography (CT) or Magnetic Resonance Imaging (MRI) data. However, any other type of medical image data that can be processed by a CAD system can be used. The medical image data are then feed into a CAD system for processing by a CAD algorithm in step 20. Once the CAD algorithm has processed the medical image or set of images, the medical image data, pixel similarity scores and associated detected (thresholded) pixels defined by the CAD output as CAD-detected regions are retrieved. Using the CAD outputs and the medical image data, the medical image is produced for inspection on a softcopy (e.g., a review workstation or a computer screen) or hardcopy (e.g., printed on paper) in step 30. The CAD-detected regions of concern indicated by the CAD algorithm are enhanced in a manner such that the user, or radiologist, can quickly and easily find all regions of interest.

All the pixels in the CAD-processed medical image are selectively enhanced and/or suppressed in accordance to a similarity score. The basis for the enhancement alteration of the native pixel values on the CAD-processed medical image can be particular attributes associated with relevant observables, such as, for example, the foci on converging lines, regions believed to contain multiple microcalcifications (i.e., microcalcification clusters), or any other attributes known in the art.

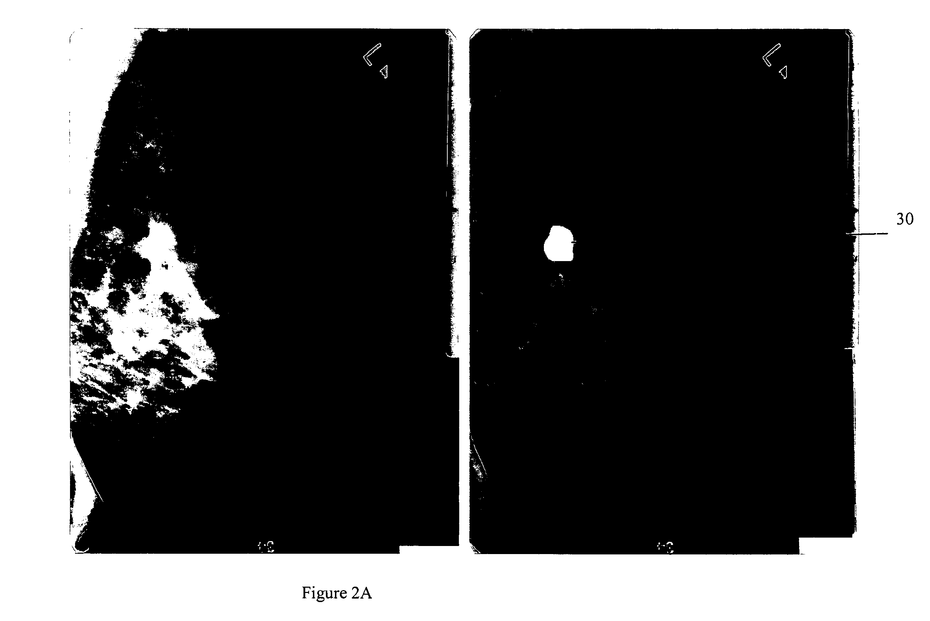

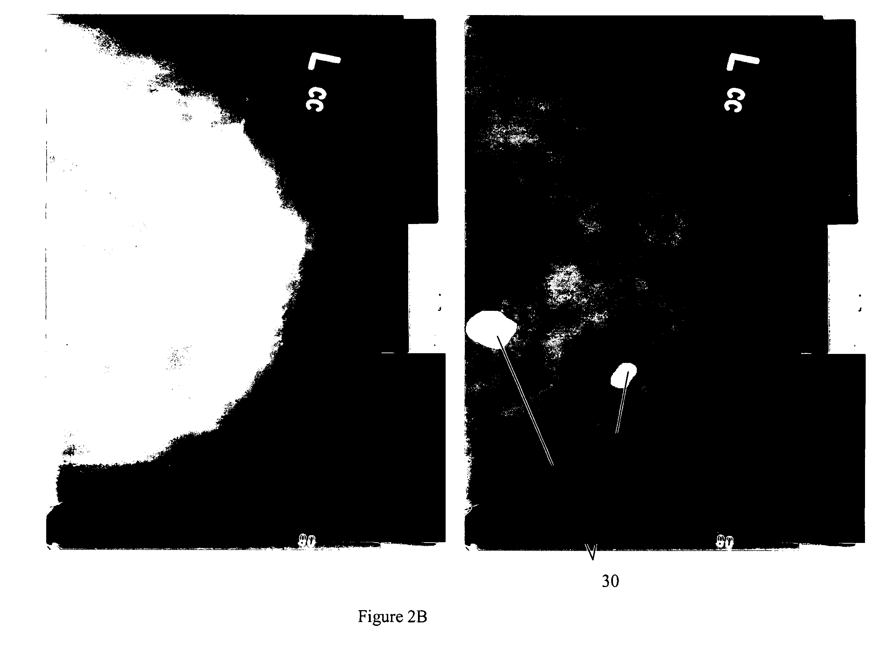

In one embodiment, illustrated in Figs. 2A and B, a region 30 that encompasses the CAD-detected region will be brightened and the contrast within the encompassed region 30 will be enhanced significantly on the displayed or printed processed medical image. The region 30 could be a geometric shape such as, for example, an oval or circle, or any other shape that fully encompasses the CAD-detected region. The region 30 could also be an arbitrary shape that is meant to depict the shape of the detected region. At the same time, all other image data on the displayed or printed medical image may be darkened or faded such that the enhanced encompassed region 30 clearly stands out from the rest of the image. Several enhanced encompassed regions 30 can be shown on the displayed or printed medical image as illustrated in Fig. 2B. In this embodiment, the image detail surrounding the CAD-detected region is enhanced rather than obscured by the CAD detection allowing the user to understand why the region was marked. At the same time, the non-important areas (i.e., the areas not containing the CAD-detected region and its associated encompassed region 30) are darkened, muted, or dimmed, so that the user can concentrated on the CAD-detected regions.

In another embodiment, the regions of concern indicated by the CAD algorithm(s) are enhanced in a manner such that the user will quickly and easily find all regions of interest. The attributes of the image region that caused it to be detected by the CAD algorithm may be enhanced to clearly standout from the rest of the image. This can be accomplished by selectively enhancing or suppressing pixels inside and/or outside a specific region. In one embodiment, pixels associated with the regions detected by the CAD would be brightened. Alternatively, the pixels located outside the region of interest detected by the CAD algorithm could be darkened. In yet another embodiment, the pixels associated with the CAD-detected region would be brightened while the pixels falling outside the region could be darkened. Secondary annotations based on thresholding a similarity metric may be used to cue the user to discrete image locations. In all cases the degree of enhancement or de-emphasis of surrounding tissue in the medical image may be based on application of a similarity metric.

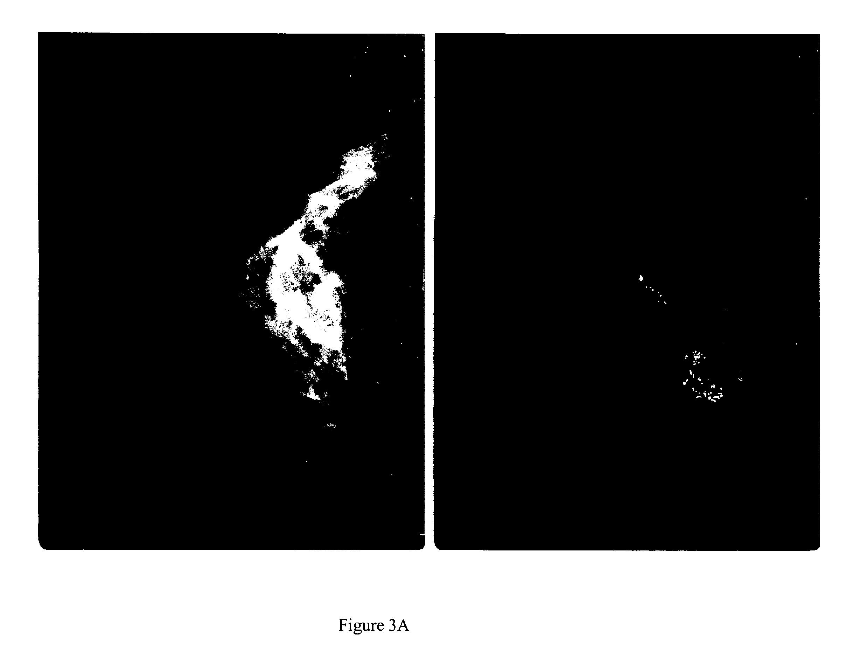

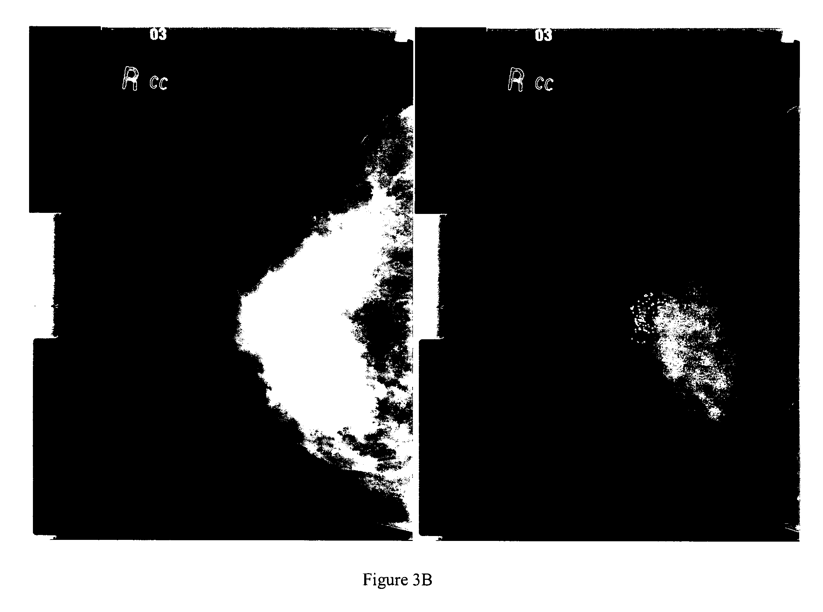

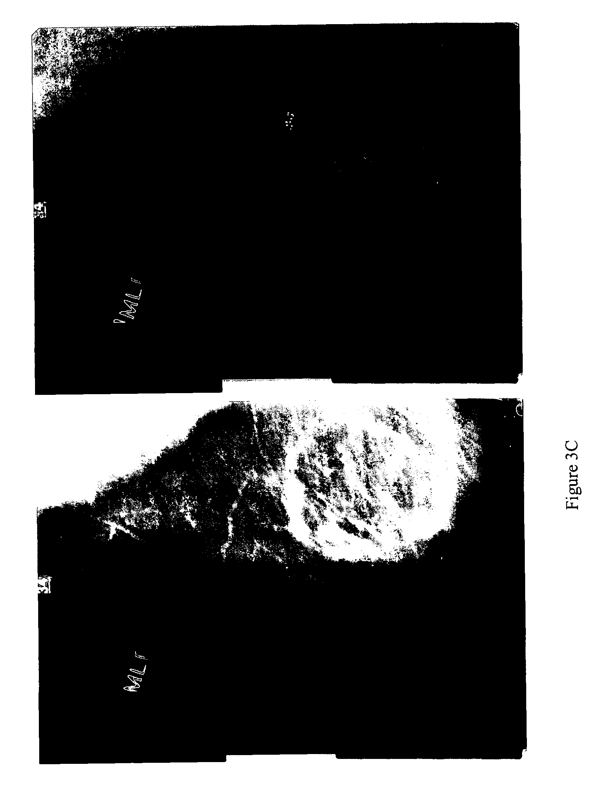

For example, in mammography the CAD algorithms are designed to detect and/or enhance several image attributes associated with indications of different types of cancer including circumscribed masses, spiculated masses, and clusters of microcalcifications. Each of these indicators of cancer have unique attributes. Spiculated masses have radiating lines (or spicules) emanating from a central location. Circumscribed masses have distinct margins and are denser, and therefore brighter, than surrounding tissue. Microcalcifications are small calcium deposits which are brighter than surrounding tissue, like a bunch of little specs, or dots. Depending on what the specific nature of the CAD algorithm(s) used for detecting and/or enhancing, the enhancement for a region and/or pixels could be customized to indicate a specific image attribute. For spiculated masses the linear structures could be enhanced. For circumscribed masses, the margins and central dense area could be enhanced. For microcalcifications, the tiny specs indicating the calcium deposits could be enhanced to make them clearly obvious using operations such as matched filters and/or morphological measurements as illustrated in Figs. 3A-C. The idea of this type of enhancement is to over-emphasize the image features the CAD algorithm found suspicious so that the user would better understand what caused the algorithm to point out a particular region.

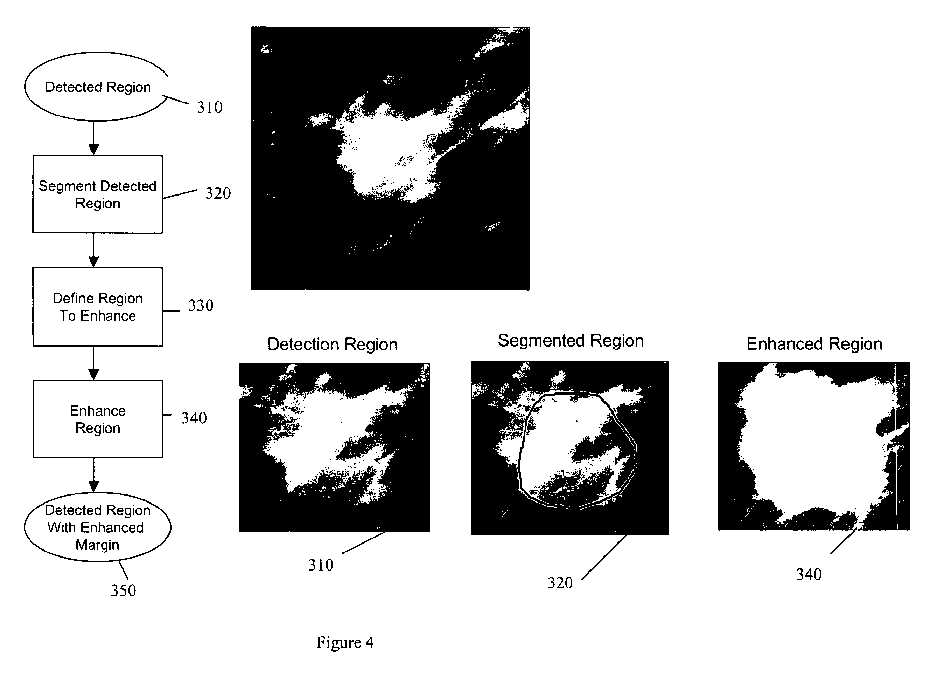

Fig. 4 illustrates the steps for detecting and enhancing a region with margins for a circumscribed mass. In the first step 310, the suspicious region is detected as a circumscribed mass. The region is then segmented in step 320 and defined in order to enhance the margin in the region in step 330. The margin in the region is then enhanced in step 340. The enhanced region is then displayed with the enhanced margin in step 350.

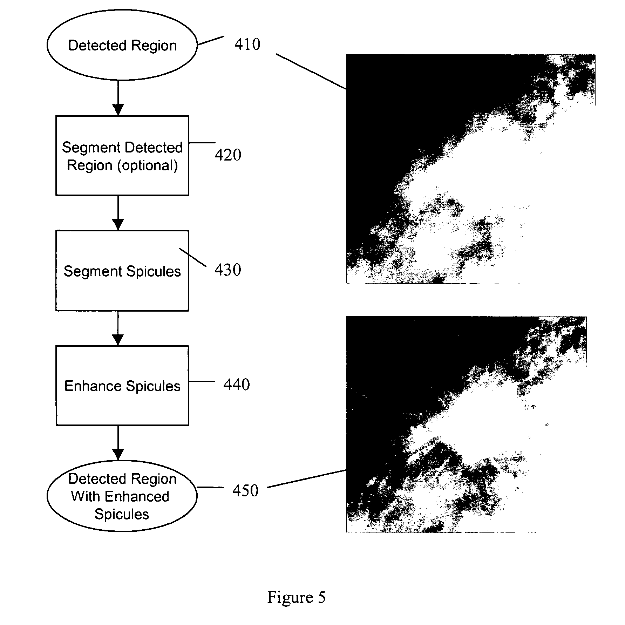

Fig. 5 illustrates the steps for detecting and enhancing a region with spicules for a spiculated mass. In the first step 410, the suspicious region is detected as a spiculated mass. The region may be optionally segmented in step 420. The spicules are then segmented in step 430. The spicules in the region are then enhanced in step 440. The region of the spiculated mass is then displayed with the enhanced spicules in step 450.

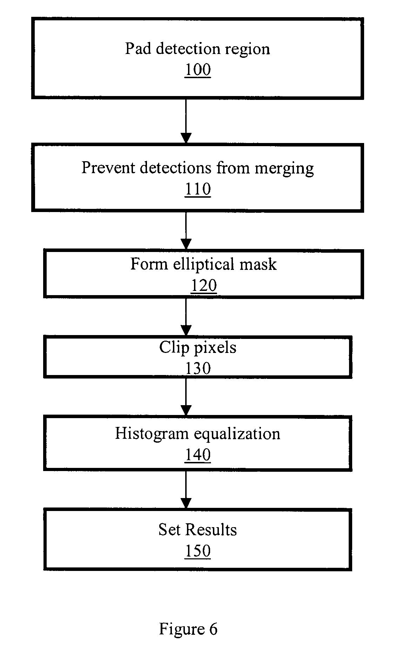

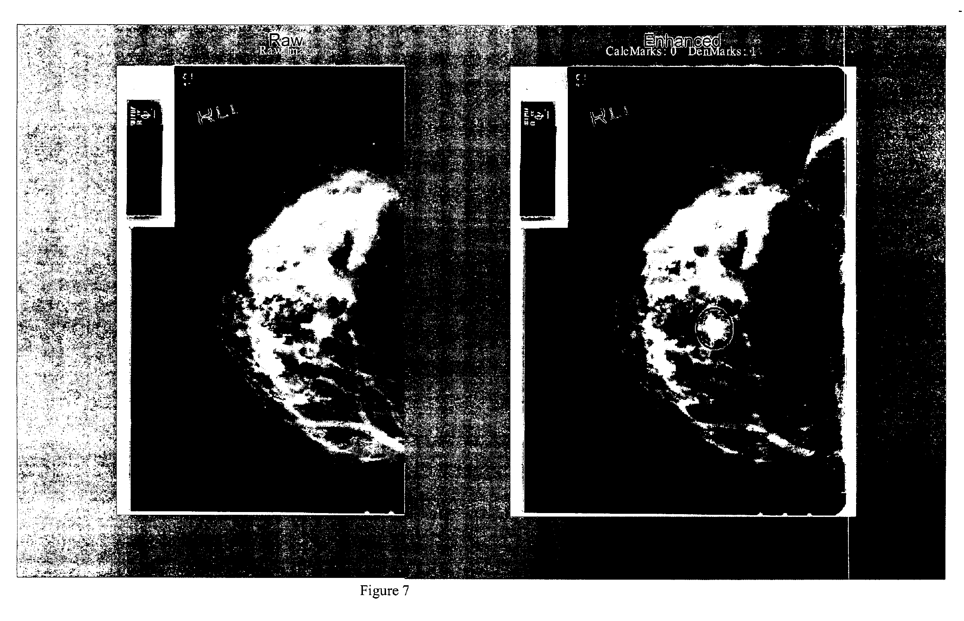

In images that contain both masses and microcalcifications, the mass detection regions are enhanced first. This order ensures that the microcalcifications that are located on masses will be clearly visible. Figure 6 illustrates the steps involved in a mass enhancement. In step 100, the detection region is padding by a bounding box. Step 110 ensures that the individual padded mass detections do not merge. By preventing the mass detections from merging ensures that only the mixed lesions will generate overlapping ellipses. An elliptical mask from the padded bounding box of the mass detection is formed in step 120. In preparation for histogram equalization, the brighter and dimmer pixels are clipped in step 130. Histogram equalization is performed in step 140. In this step, the pixels that are very bright are excluded from the histogram. For example, any chest-wall masses adjacent to off-film content are excluded. Finally, in step 150, the results are set. Figure 7 is an example of a single mass enhancement on a mammography image.

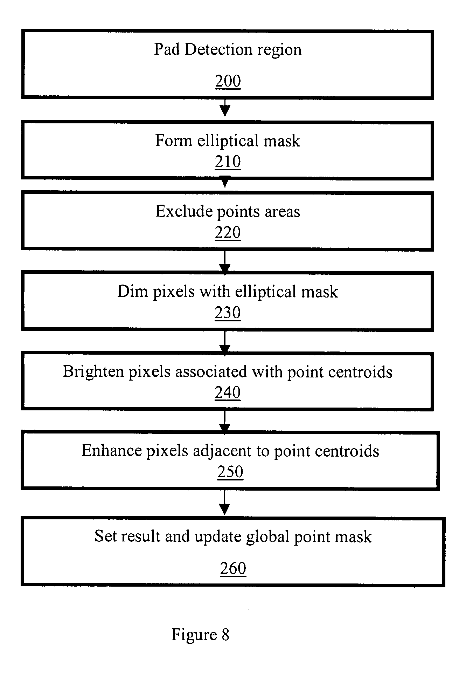

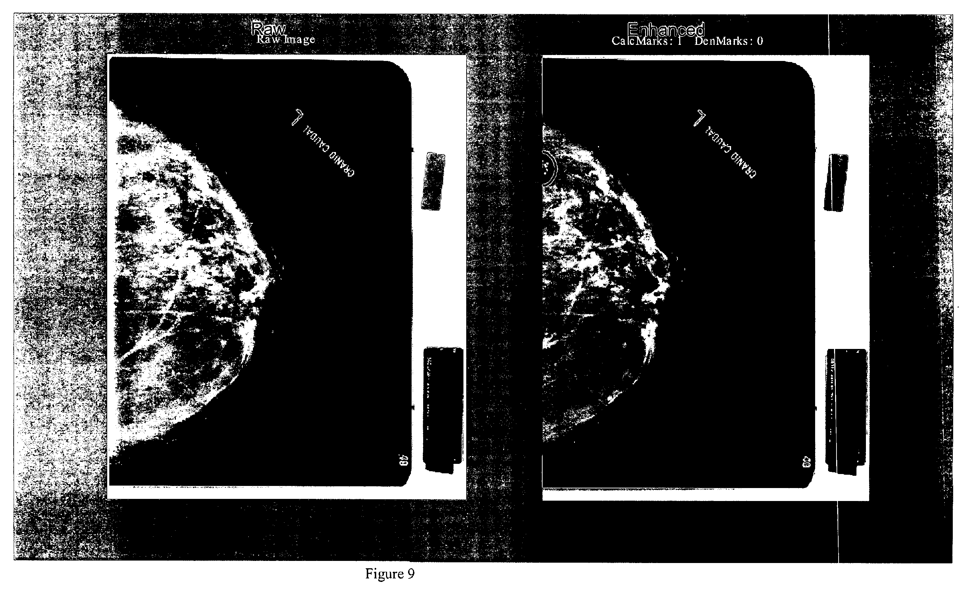

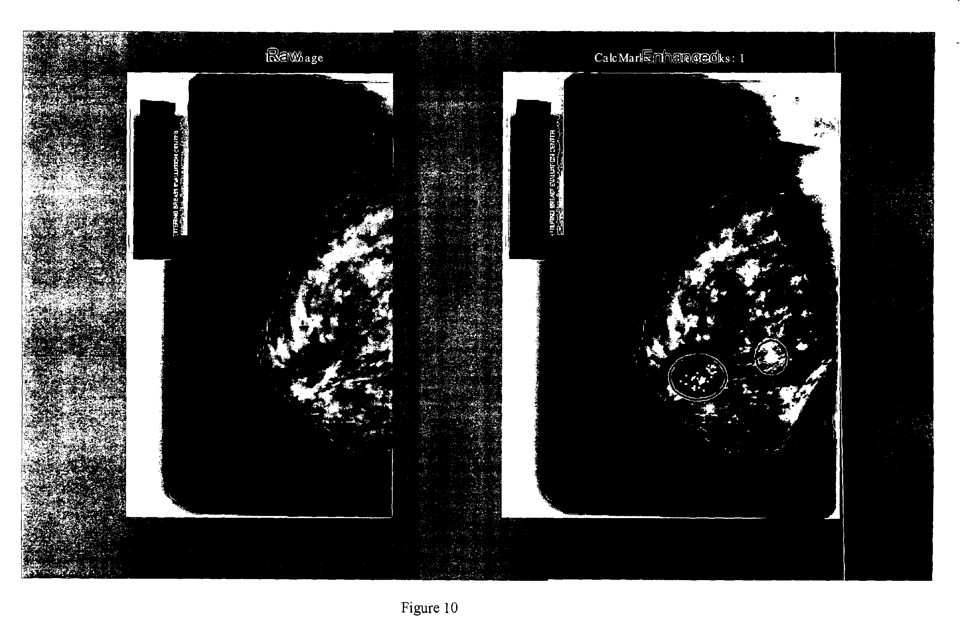

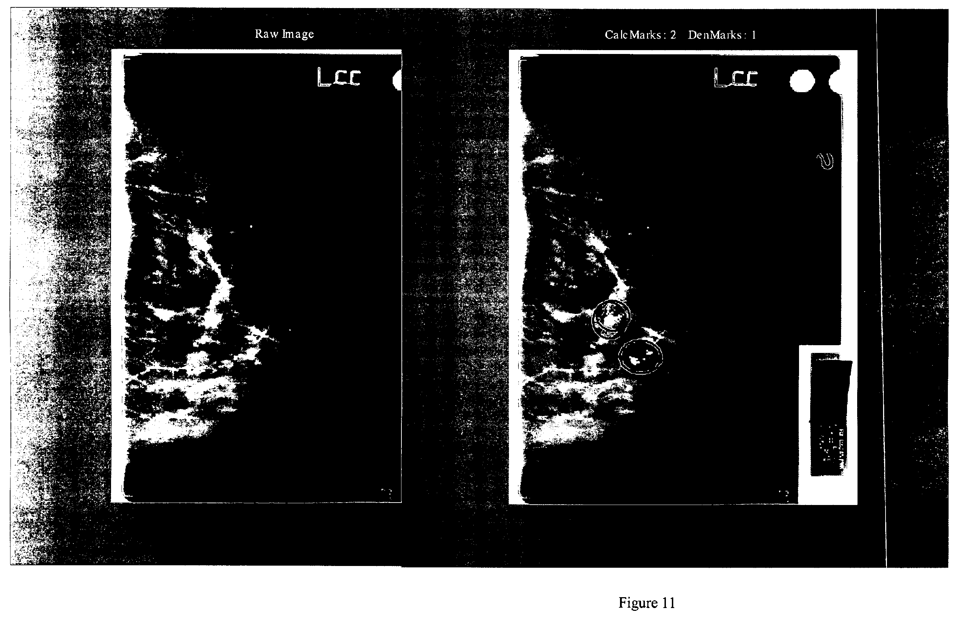

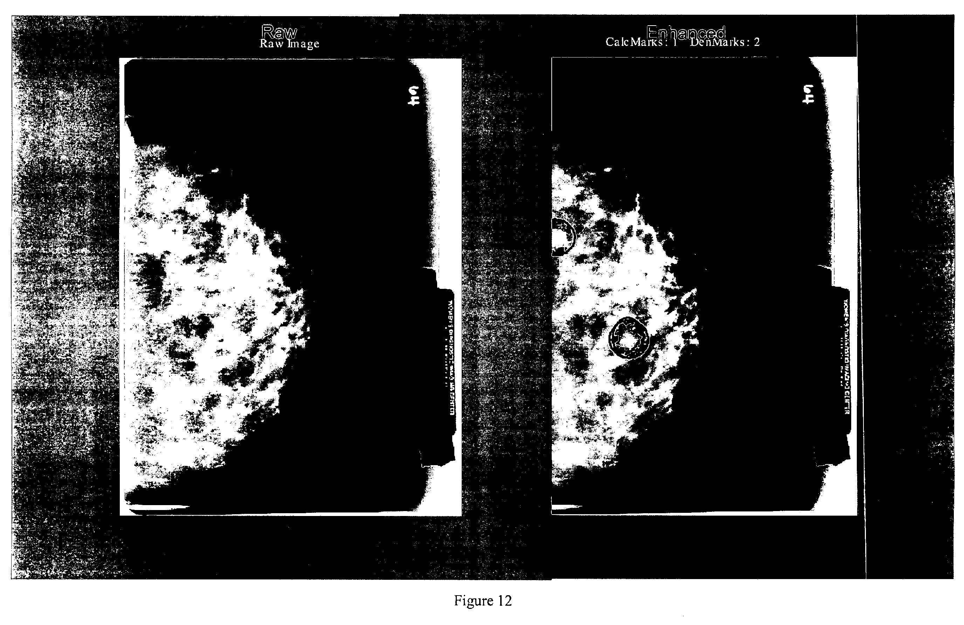

Figure 8 illustrates the steps involved in a microcalcification enhancement. In step 200, the detection region is padding by a bounding box. An elliptical mask from the padded bounding box of the microcalcification detection is formed in step 210. Points areas that may be enhanced from a neighboring cluster are excluded in step 220. This exclusion ensures that points from neighboring areas are not turned off by the padding. The pixels within the elliptical mask are dimmed by scaling the intensity values in step 230. The pixels may be scaled by 75% of the current value. To ensure that the microcalcifications are clearly visible, the maximum possible value under a mask equals 75% of 255 or 191. In step 240, the pixels associated with the point centroids are brightened. In other words, the pixels are set to the maximum brightness of 255. The pixels adjacent the point centroids are also enhanced in step 250. An eight-connected neighborhood is used to determine adjacent pixels. The amount of enhancement is based on comparing neighbor intensity values to the centroid value. The neighboring intensity value is determined to be the neighbor intensity divided by the centroid intensity multiplied by 255. The neighboring intensity values are clipped to a maximum of 255. Finally, in step 260, the results are set. A global point mask is also updated to keep track of enhanced point regions if there is more than one cluster on the image. Figure 9 is an example of a single microcalcification enhancement on a mammography image. Other examples of detection enhancements include Figure 10 which is an example of enhancements of a non-overlapping single microcalcification detection and a single mass detection on a mammography image. Figure 11 which is an example of enhancements of a single microcalcification detection and a microcalcification detection on top of a mass detection on a mammography image and Figure 12 which is an example of enhancements of a single mass detection and a microcalcification detection on top of a mass detection on a mammography image.

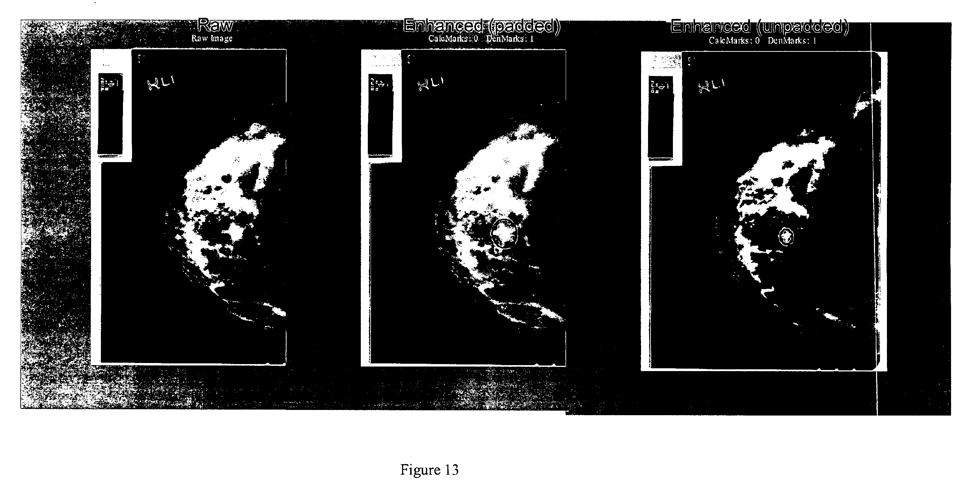

In addition, the user may increase or decrease the size of the region to be enhanced on the display. The user may manually alter the size by using a computer mouse, a computer touch screen, a pen stylus, a button on the display, or by any other method known to a practitioner of the art. Fig. 13 illustrates enhancing a mass detection by padding or not padding a mass detection.

In yet another embodiment, when the medical imagery is to be displayed on a workstation display, or computer monitor, after CAD algorithm processing (i.e., for softcopy review), the user would have the option to animate or toggle the CAD-detected regions, the enhanced regions that encompass the CAD-detected region and/or pixels associated with the CAD-detected region on or off. This option would allow the user to toggle on the CAD-detected regions, enhanced encompassed regions and/or pixels so that the regions clearly stand out from their surrounding background on the medical image. For example, the CAD-detected regions and/or pixels (or the background around the regions) could alternately brighten and fade as the user toggles the enhancements on or off in order to increase the visual conspicuity of the regions and/or pixels. This option would allow the user to start, stop, or turn the CAD-detected region's enhancements on or off.

Finally, in another embodiment, the CAD-detected image regions could be selectively segmented (chipped or cropped) and displayed in isolation to further improve the visibility of image attributes known to be associated with cancer. These chipped regions could be toggled on and off on a workstation monitor based on user invoked controls. To aid the user in associating chipped regions with specific image locations, in one embodiment, an annotation map identifying the location of the chip(s) in the medical image would be provided to the user. In another embodiment, a known protocol for chip layout specified (e.g., raster scan format) could be provided to the user. Alternatively, both the annotation map and known protocol could be provided to the user to associated the chipped region with the overall medical image.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

Having described the invention in detail and by reference to specific embodiments thereof, it will be apparent that modifications and variations are possible without departing from the scope of the invention defined in the appended claims. More specifically, although some aspects of the present invention are identified herein as preferred or particularly advantageous, it is contemplated that the present invention is not necessarily limited to these preferred aspects of the invention.

What is claimed is:

## Claims

1. A method for enhancing the display of computer-aided detection system findings on a medical image, the method comprising:
obtaining the medical image;
processing the medical image by a computer-aided detection system algorithm, wherein the computer-aided detection system algorithm detects regions of interest on the medical image and enhances the regions without obscuring the underlying structures of the medical image; and
outputting the processed medical image with the detected regions of interest enhanced on the processed medical image.

2. The method of claim 1, wherein the medical image comprises an image that may be processed by a computer-aided detection system.

3. The method of claim 1, wherein the medical image comprises a mammography X-ray image, a lung X-ray image, a colonoscopy image or a combination of medical images.

4. The method of claim 1, wherein the region detected by the computer-aided system algorithm is displayed on the medical image in a shape that fully encompasses the region of interest.

5. The method of claim 4, wherein enhancing the region involves brightening the shape encompassing the computer-aided detection system algorithm detected regions of interest on the medical image.

6. The method of claim 4, wherein enhancing the region involves dimming the area outside of the shape encompassing the computer-aided detection system algorithm detected regions of interest on the medical image.

7. The method of claim 4, wherein enhancing the region involves brightening shape encompassing the computer-aided detection system algorithm detected regions of interest and dimming the area outside of the shape encompassing the computer-aided detection system algorithm detected regions of interest on the medical image.

8. The method of claim 1, wherein enhancing the region involves brightening the pixels within the computer-aided detection system algorithm detected regions of interest on the medical image.

9. The method of claim 1, wherein enhancing the region involves dimming the pixels outside of the computer-aided detection system algorithm detected regions of interest on the medical image.

10. The method of claim 1, wherein enhancing the region involves brightening the pixels within the computer-aided detection system algorithm detected regions of interest and dimming the pixels outside of the computer-aided detection system algorithm detected regions of interest on the medical image.

11. The method of claim 1, wherein enhancing the region involves brightening or dimming pixels on the medical image based on a similarity metric.

12. The method of claim 1, wherein enhancing the region on the medical image further comprises:
annotating the medical image to cue a user to discrete locations on the medical image, wherein the annotations are based on thresholding a similarity metric.

13. The method of claim 1, wherein enhancing the region is based on the computer-aided detection system algorithm used and customized to a specific medical image attribute a user wishes to emphasize.

14. The method of claim 1, wherein enhancing the region involves over-emphasizing unique characteristics of the computer-aided detection system algorithm detected regions of interest on the medical image based on the computer-aided detection system algorithm used.

15. The method of claim 14, wherein over-emphasizing unique characteristics of the computer-aided detection system algorithm detected regions of interest on the medical image enhances linear structures if the detected region of interest is a spiculated mass.

16. The method of claim 14, wherein over-emphasizing unique characteristics of the computer-aided detection system algorithm detected regions of interest on the medical image enhances margins and central dense areas if the detected region of interest is a circumscribed mass.

17. The method of claim 14, wherein over-emphasizing unique characteristics of the computer-aided detection system algorithm detected regions of interest on the medical image enhances tiny dots representing small calcium deposits if the detected regions of interest are microcalcifications.

18. The method of claim 1, wherein enhancing the region, further comprises:
enhancing mass structures before enhancing microcalcifications.

19. The method of claim 18, wherein enhancing mass structures comprises the steps of:
padding the computer-aided detection system algorithm mass detections on the medical image by a bounding box;
ensuring the padded bounding boxes of individual mass detections do not merge;
forming an elliptical mask from the padded bounding box of the mass detection;
clipping the brightest and dimmest pixels from the padded bounding box of the mass detection;
performing histogram equalization on the padded bounding box of the mass detection; and
setting the results of the histogram equalization.

20. The method of claim 18, wherein enhancing microcalcification structures comprises the steps of:
padding the computer-aided detection system algorithm microcalcification detections on the medical image by a bounding box;
forming an elliptical mask from the padded bounding box of the microcalcification detection;
excluding enhanced microcalcification point areas from neighboring clusters;
dimming pixels within the elliptical mask by scaling the intensity values of the pixels;
brightening pixels within the elliptical mask associated with point centroids;
enhancing pixels adjacent to the point centroids; and
setting the results.

21. The method of claim 20, wherein scaling is up to about 75% of the maximum intensity value of the pixels.

22. The method of claim 20, wherein brightening pixels within the elliptical mask associated with point centroids is brightened up to the maximum brightness of the pixels.

23. The method of claim 20, wherein the pixels adjacent to the point centroids are determined by an eight-connected neighborhood.

24. The method of claim 20, wherein the amount pixels adjacent to the point centroids are enhanced is determined by comparing the intensity values of the neighboring pixels to the point centroid intensity value.

25. The method of claim 20, wherein enhancing microcalcification structures further comprising the step of:
updating a global point mask to keep track of enhanced point regions if more than one microcalcification cluster exists on the medical image.

26. The method of claim 1, wherein outputting the processed medical image is outputted to a printer.

27. The method of claim 1, wherein outputting the processed medical image is outputted to an electronic display.

28. The method of claim 27, further comprising:
toggling the display on the electronic display of the computer-aided detection system algorithm detected regions of interest on and off.

29. The method of claim 1, further comprising:
altering the size of the computer-aided detection system algorithm detected region of interest displayed on the processed medical image.

30. The method of claim 29, wherein altering the size of the computer-aided detection system algorithm detected region of interest displayed is controlled manually through the use of user-invoked controls.

31. The method of claim 30, wherein altering the user-invoked controls comprise a computer mouse, a touch screen, a pen stylus, a display button, or combinations thereof.

32. A method for enhancing the display of computer-aided detection system findings on a medical image, the method comprising:
obtaining the medical image;
processing the medical image by a computer-aided detection system algorithm, wherein the computer-aided detection system algorithm detects regions of interest on the medical image and enhances the regions without obscuring the underlying structures of the medical image based on a specific medical attribute a user wants to emphasize; and
outputting the processed medical image to an electronic display.

33. The method of claim 32, further comprising:
starting the display of the computer-aided detection system algorithm detected and enhanced regions of interest on the electronic display.

34. The method of claim 32, further comprising:
stopping the display of the computer-aided detection system algorithm detected and enhanced regions of interest on the electronic display.

35. The method of claim 32, further comprising:
toggling the display on the electronic display of the computer-aided detection system algorithm detected and enhanced regions of interest on and off.

36. The method of claim 35, wherein toggling the display on the electronic display of the computer-aided detection system algorithm detected regions of interest on brightens the pixels within the computer-aided detection system algorithm detected regions of interest to stand out from the rest of the medical image.

37. The method of claim 35, wherein toggling the display on the electronic display of the computer-aided detection system algorithm detected regions of interest off dims the pixels within the computer-aided detection system algorithm detected regions of interest to no longer stand out from the rest of the medical image.

38. The method of claim 32, further comprising:
segmenting the computer-aided detection system algorithm detected and enhanced regions of interest from the medical image; and
displaying the segmented computer-aided detection system algorithm detected and enhanced regions of interest in isolation from the medical image on the electronic display.

39. The method of claim 38, further comprising:
annotating the location of the segmented computer-aided detection system algorithm detected and enhanced regions of interest within the overall medical image on a map displayed on the electronic display.

40. The method of claim 38, further comprising:
providing a known protocol for the layout of the segmented computer-aided detection system algorithm detected and enhanced regions of interest on the electronic display.

41. The method of claim 38, further comprising:
providing a known protocol for the layout of the segmented computer-aided detection system algorithm detected and enhanced regions of interest; and
annotating the location of the segmented computer-aided detection system algorithm detected and enhanced regions of interest within the overall medical image on a map displayed on the electronic display.

42. The method of claim 38, further comprising:
toggling the display of the segmented computer-aided detection system algorithm detected and enhanced regions of interest on or off using user-invoked controls.

43. A system for enhancing the display of computer-aided detection system findings on a medical image, the system comprises:
a medical image;
a computer-aided detection algorithm which processes the medical image to detect and enhance regions of interest on the medical image without obscuring the underlying structures on the medical image; and
an output device for displaying the computer-aide detection algorithm detected and enhanced regions of interest on the medical image.

44. The system of claim 43, wherein the computer-aided detection algorithm enhances the pixels within the medical image based in accordance with a similarity score.

45. The system of claim 43, wherein the computer-aided detection algorithm enhances the pixels within the medical image based on particular attributes associated with computer-aided detection observables.

46. The system of claim 43, wherein the computer-aided detection algorithm enhances the regions of interest by brightening pixels within the regions of interest.

47. The system of claim 43, wherein the computer-aided detection algorithm enhances the regions of interest by dimming the pixels outside the regions of interest on the medical image.

48. The system of claim 43, wherein the computer-aided detection algorithm enhances the regions of interest by brightening pixels within the regions of interest and dimming the pixels outside the regions of interest on the medical image.

49. The system of claim 43, wherein the output device is a printer.

50. The system of claim 43, wherein the output device is an electronic display.

51. The system of claim 50, wherein the output display toggles the display of the computer-aided detection system algorithm enhanced regions of interest on and off on the medical image.

52. A method for enhancing the display of computer-aided detection system findings on a medical image, the method comprising:
obtaining the medical image;
enhancing regions of interest detected by computer-aided detection system algorithm on the medical image without obscuring the underlying structures of the medical image by brightening the regions of interest and dimming the rest of the medical image ; and
outputting the processed medical image with the detected regions of interest enhanced on the processed medical image.

53. The method of claim 52, wherein enhancing regions of interest is based on the computer-aided detection system algorithm used and customized to a specific medical image attribute the user wishes to emphasize.

54. The method of claim 52, wherein enhancing regions of interest further comprises:
over-emphasizing unique characteristics of the computer-aided detection system algorithm detected regions of interest on the medical image based on the computer-aided detection system algorithm used.

55. A method for enhancing the display of computer-aided detection system findings on a medical image, the method comprising:
obtaining the medical image;
enhancing regions of interest detected by computer-aided detection system algorithm on the medical image without obscuring the underlying structures of the medical image;
annotating the medical to cue a user to discrete locations on the medical image; and
outputting the processed medical image with the detected regions of interest enhanced on the processed medical image.

56. A method for enhancing the display of computer-aided detection system findings on a medical image, the method comprising:
obtaining the medical image;
enhancing regions of interest detected by a computer-aided detection system algorithm without obscuring the underlying structures of the medical image based on a specific medical attribute a user wants to emphasize; and
outputting the processed medical image to an electronic display, wherein the output to the electronic display of the enhanced regions of interest is toggled on and off by user-invoked controls.

57. A method for enhancing the display of computer-aided detection system findings on a medical image, the method comprising:
obtaining the medical image;
enhancing regions of interest detected by a computer-aided detection system algorithm on the medical image without obscuring the underlying structures of the medical image;
segmenting the enhanced regions of interest from the medical image; and
displaying the enhanced regions of interest in isolation from the rest of the medical image on an outputted medical image.

58. The method of claim 57, further comprising:
providing a known protocol for the layout of the segmented enhanced regions of interest; and
annotating the location of the segmented enhanced regions of interest within the overall medical image on a map displayed on the outputted medical image.
